# EUROPEAN PATENT APPLICATION

(11) **EP 4 822 705 A1**
(43) Date of publication of application: **14.10.2026**
(21) Application number: 25169004.6
(22) Date of filing: 08.04.2025
(51) Int. Cl.: G16H 15/00, A61B 1/00, G06T 7/00, G16H 30/40, G16H 50/20, G16H 50/30, G16H 50/70

(54) **PERSONALIZED ORAL CARE GUIDANCE SYSTEM**

(71) Applicant: Koninklijke Philips N.V., 5656 AE Eindhoven (NL)
(72) Inventor: BIRADAR, Mayuri, Eindhoven (NL); KUNAPALLI, Manoj Kiran, Eindhoven (NL); DEVANAHALLI BALAKRISHNA, Naveen, Eindhoven (NL)
(74) Representative: Philips Intellectual Property & Standards

(57) **Abstract**

Personalized oral care guidance is provided by capturing a dental image of a subject at an end user device, identifying oral health issues using a machine-learning model, and generating a mouth map with the issues. The mouth map can indicate the location and severity of oral health problems, such as cavities, gum disease, or tooth misalignment. The device displays the mouth map on a screen, allowing users to visualize their oral health and receive tailored guidance on how to address these issues. An oral care device acts on the insights based on the image analysis and adjusts the settings for real-time brushing.

## Description

### TECHNICAL FIELD

This disclosure relates to personalized oral care technology, and more specifically to personal oral care guidance systems using dental image processing.

### BACKGROUND

Personal oral health care has traditionally relied on basic tools and methods, such as manual brushing, flossing, and periodic dental visits.

Despite these traditional approaches, current dental care products and technologies often lack integration and fail to provide real-time, actionable insights for users.

For example, US 2021/0201687 A1 discloses a method of providing tooth-brushing guide information. A user terminal analyzes a captured image of a user, recognizes the face of the user, and identifies landmarks of the face. The user terminal generates a personalized dental model of the user using the landmarks. The user terminal provides a virtual dental image by augmented reality using the dental model and provides tooth-brushing guide information using the dental model.

However, dental care applications like this offer limited feedback, focusing mainly on user behavior tracking without in-depth analysis or advanced diagnostics. This limited feedback can lead to inadequate oral health management, potentially resulting in suboptimal outcomes.

Furthermore, X-ray imaging, while valuable, is typically confined to periodic visits to the dentist, offering insights that are not integrated with daily oral care practices. The lack of integration and real-time feedback hinders users from making informed decisions about their oral health.

It is therefore an objective of the present disclosure to provide a more comprehensive and integrated approach to managing oral health, overcoming at least in part the limitations of current dental care products and technologies.

### SUMMARY OF THE DISCLOSURE

The above and other objectives may be achieved by the subject-matter defined in the independent claims. Advantageous modifications of embodiments of the present disclosure are defined in the dependent claims as well as in the description and the drawings.

One aspect of the present disclosure relates to a method of providing personalized oral care guidance. The method may be computer-implemented. The method, i.e., some or all method steps, may be performed by an application ("app") that executes on a device, in particular an end user device, such as a smartphone, smart display, smart mirror, or the like, of a subject.

It may be provided that the method comprises receiving a dental image of the subject. Preferably, the dental image has been captured by an oral scanning device also commonly referred to as an intraoral scanner, which is a device used in dentistry to capture digital images of the inside of the mouth. The dental image can be taken by a healthcare professional such as a dentist, in which case the subject is a patient of the healthcare professional. Alternatively, the dental image can be taken by the subject themselves, e.g,, by using a home intraoral scanner or even the subject's end user device (in this case, the end user device may represent the oral scanning device). The dental image may be an X-ray image, a CT image, an MRI image, a photo, a video, or the like. Regardless of the exact image modality, the dental image should provide a detailed view of the subject's teeth, gums, jaw and/or tongue, so that any oral health issues, such as cavities, gum disease or misalignment, can be revealed.

It may be provided that the dental image of the subject is received at an end user device of the subject, such as a smartphone. The end user device may receive the dental image, e.g., when the subject imports the dental image into an application on the end user device. As an alternative, the end user device may directly fetch the dental image from a remote source, such as a server operated by the healthcare professional.

It may be provided that the method comprises identifying one or more oral health issues in the dental image. The identifying step may be performed using an image-processing machine-learning model. This way, the input dental image can be analyzed comprehensively, and a detailed report of the subject's dental conditions can be generated. The identifying step may be performed by the end user device, in particular by the application executing thereon.

It may be provided that the method comprises generating a mouth map of the subject which indicates the one or more oral health issues. It may be provided that the method comprises displaying the mouth map with the indicated one or more oral health issues on a display of the end user device. The mouth map may comprise a visual representation of the subject's mouth. Preferably, the mouth map is a tooth-by-tooth mouth map. Preferably, the mouth map is customized to the subject's mouth. The report, i.e. the identified one or more oral health issues, can be used to create a personalized mouth map of the subject, ideally tooth by tooth. The mouth map can highlight areas that need special attention based on the report, such as cavities, plaque build-up, gum disease, or the like.

This way, several advantages can be achieved in the realm of personal oral care, including without limitation:
1. More Effective Oral Care: Many subjects do not brush their teeth effectively, missing key areas or not brushing for long enough. The mouth map, with its highlighting of areas that need special attention based on any issues observed in the dental image, can help subjects improve their brushing techniques and thus eventually their oral health.
2. Improved Personalization: Traditional oral care advice is often generic and not tailored to the subject's specific needs. The proposed method can provide personalized guidance based on the subject's unique oral health profile as determined from the dental image.
3. Lack of Engagement: Traditional methods of oral health education can be dry and non-engaging. The use of a visually engaging, personalized mouth map can make the process more engaging, encouraging subjects to take a more active role in their oral health.

It may be provided that the one or more oral health issues comprise one or more of an indication of a cavity in one or more teeth, preferably indicated for the one or more specific teeth, an indication of plaque build-up over one or more teeth, preferably indicated for the one or more specific teeth, an indication of a gum disease, such as inflammation, preferably indicated for one or more specific teeth, an indication of a misalignment, such as a gap, preferably indicated for one or more specific teeth, an indication of tongue hygiene, or any combination thereof.

This way, a comprehensive report including the one or more oral health issue can be identified and indicated on the mouth map, which in turn can further improve the advantages mentioned above.

It may be provided that the method further comprises displaying oral care guidance, preferably on the mouth map, while the subject is performing a personal oral care routine, such as toothbrushing, flossing, and the like. The oral care guidance may comprise one or more focus areas. Preferably, the oral care guidance is personalized based on the dental image analysis. The oral care guidance can be displayed on a display of the subject's end user device. In one possible implementation, the oral care guidance is displayed in an augmented reality view. Such augmented reality view may be overlaid onto the mouth map and/or the dental image. Accordingly, a personalized mouth map can be created to guide the subject in real time as they brush their teeth. The mouth map can highlight the areas of focus and provide live feedback to ensure that the subject is brushing these areas effectively.

Such a real-time display of oral care guidance while the subject brushes their teeth enables more effective oral care. Many subjects do not brush their teeth effectively, missing key areas or not brushing for long enough. The proposed live (augmented reality) guidance can help subjects improve their brushing techniques. It allows for a highly personalized and effective brushing experience, tailored to the subject's specific dental health needs. The real-time oral care guidance can also improve oral health understanding. Many subjects find oral or dental health concepts abstract and difficult to understand. By using an easy-to-understand visualization, such as with augmented reality, to visualize oral health and progress, these concepts are made more tangible and understandable.

It may be provided that the method comprises providing the one or more oral health issues to an electronic personal oral care device to cause the personal oral care device to adapt at least one oral care setting. The at least one oral care setting may comprise speed, pressure, direction, oscillation, or the like. Adapting at least one oral care setting may comprise utilizing location sensing. For example, when the personal oral care device recognizes that it is operating in an area of the subject's mouth where there is a gum issue, the personal oral care device can decrease the speed of the brushing movement. The personal oral care device may automatically adjust its brushing techniques for each subsegment of the subject's mouth, such as for each tooth, to ensure effective cleaning. Adapting at least one oral care setting may also comprise giving haptic feedback to the subject when a change of a setting is happening, so that the subject is aware of this. The one or more oral care settings may be updated every time a new dental image is received and analyzed, as will be explained further below.

Many subjects do not brush their teeth effectively, missing key areas or not brushing for long enough. A smart toothbrush auto adaption technique as explained above can help improve the dental health of the subject.

It may be provided that the method comprises generating a personalized oral care routine tutorial based on the one or more oral health issues. This can further improve oral health because the subject is effectively taught the right routine personalized to their situation.

It may be provided that the dental image is a first dental image, in which case the method further comprises receiving a second dental image of the subject which was taken later than the first dental image. The method may comprise determining at least one oral care trend based on a comparison of the first dental image and second dental image. The method may comprise displaying the at least one oral care trend on a display of the end user device and/or in an augmented reality view. The at least one oral care trend may be determined using a machine-learning model, such as the image-processing machine-learning model already discussed above.

Accordingly, upon uploading a new dental image, the data can be analyzed to identify trends, highlight areas of improvement and pinpoint regions that require special attention. Augmented Reality (AR) and/or Artificial Intelligence (AI) can be leveraged to make the comparison visually engaging and easily understandable for the subject. It is often difficult for subjects to track their oral health progress over time. By comparing subsequent dental images taken at different times, the method according to this aspect provides clear visual evidence of improvement or areas that need more attention. An interactive AR and/or personalized feedback can make the process more engaging, encouraging subjects to take a more active role in their oral health. Such analysis can be repeated each time a new dental image is available, as the disclosed methods are not limited to only the two exemplary dental images mentioned above.

It may be provided that the first dental image and/or the second dental image and/or any additional dental image used in the methods comprises one or more of an X-ray image, a CT image, an MRI image, a photo, a video, or combinations thereof. Preferably, the first dental image and/or the second dental image and/or any additional dental image used in the methods shows the subject's teeth, gums, jaw, and/or tongue.

It may be provided that the machine-learning model has been trained on a training dataset. The training may comprise supervised training. The training data set may comprise a plurality of dental images labelled with oral health issues.

It may be provided that the machine-learning model executes on the end user device. Alternatively, the machine-learning model may execute on a computing device remote from the end user device and may exchange data with the end user device.

Another aspect of the present disclosure relates to a data processing system or apparatus. The data processing system or apparatus may comprise means for carrying out any combination of steps of the described methods. The data processing system or apparatus may comprise a memory and a processor. The memory may store, or be configured to store, instructions that, when executed by the processor, configure the data processing system or apparatus to carry out any combination of steps of the described methods.

Another aspect of the present disclosure relates to a computer program. Another aspect of the present disclosure relates to a non-transitory computer-readable medium having stored thereon a computer program. In both cases, the computer program may comprise instructions which, when the program is executed by a computer, such as the mentioned data processing system, cause the computer to carry out any combination of steps of the described methods. A computer program may also be referred to as a program, software, a software application, an app, a module, a software module, a script, or code. A computer program may be written in a programming language, including compiled or interpreted languages. A computer program may be deployed in any form, including as a stand-alone product or as a module, component, subroutine, or other unit suitable for use in a computing environment, such as on the mentioned data processing system.

The terms used herein should generally be construed as understood by the average person skilled in the art, unless explicitly indicated otherwise. The following explanations may guide the understanding:
Particular and preferred aspects of the present disclosure are set out in the accompanying independent and dependent claims. Features from the dependent claims may be combined with features of the independent claims and with features of other dependent claims as appropriate and not merely as explicitly set out in the claims.

The above and other characteristics, features and advantages of the present disclosure will become apparent from the following detailed description, taken in conjunction with the accompanying drawings, which illustrate, by way of example, the principles of the disclosure. This description is given for the sake of example only, without limiting the scope of the disclosure.

### BRIEF DESCRIPTION OF THE DRAWINGS

The present disclosure may be better understood by reference to the following drawings:
FIG. 1 illustrates a schematic block diagram of a system architecture in accordance with one embodiment.
FIG. 2 illustrates a flowchart of a personalized oral care guidance process in accordance with one embodiment.
FIG. 3 illustrates a more detail flowchart of a personalized oral care guidance process in accordance with one embodiment.
FIG. 4 illustrates an example of a mouth map on a graphical user interface in accordance with one embodiment.
FIG. 5 illustrates an example of an augmented reality view of a comparison of dental images in accordance with one embodiment.
FIG. 6 illustrates an example of a training image in accordance with one embodiment.
FIG. 7 illustrates a schematic block diagram of computer hardware on which embodiments of the present disclosure can be implemented.

### DETAILED DESCRIPTION

In the following, representative embodiments illustrated in the accompanying drawings will be explained. It should be understood that the illustrated embodiments and the following descriptions refer to examples which are not intended to limit the embodiments to one preferred embodiment.

In certain embodiments, a novel application ("app") is provided which leverages the power of Augmented Reality (AR) and Artificial Intelligence (AI) to provide personalized oral care guidance to subjects (users). By combining AI-driven analysis, interactive imaging and AR, the application fills the gaps left by traditional methods. The proposed application offers real-time adaptation of brushing settings, detailed analysis of the image analysis data, and thus providing a unified and intuitive system for enhancing patient care.

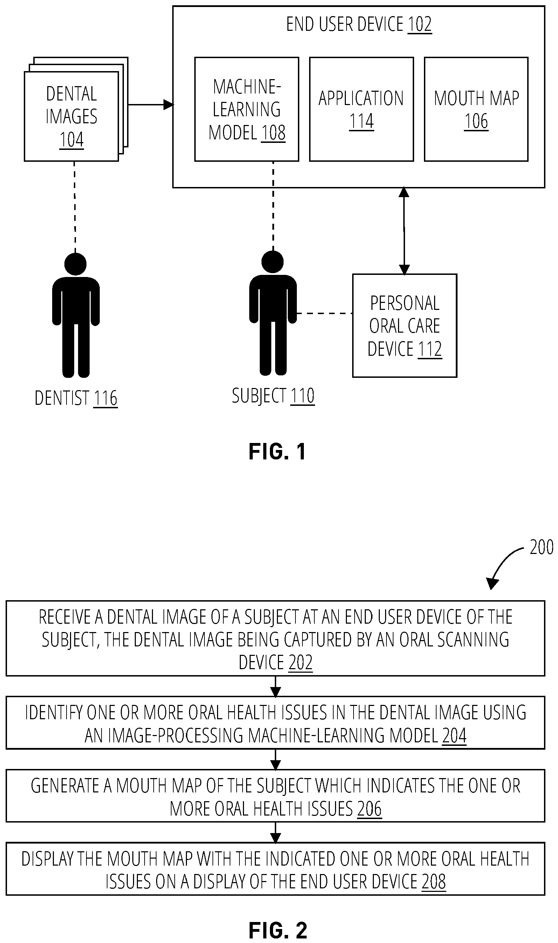

FIG. 1 illustrates a schematic block diagram of a system architecture in accordance with one embodiment. A subject 110, which is a person or end user, has an end user device 102, such as a smartphone, and a personal oral care device 112, such as an electronic toothbrush. The end user device 102 can receive dental images 104 taken by a dentist 116 or similar healthcare professional, e.g. using an oral scanning device (not shown in FIG. 1). In addition or alternatively, the end user device 102 can also receive dental images 104 taken by the subject 110 themselves, such as by using an oral scanning device or even directly using their end user device 102. The end user device 102 comprises a machine-learning model 108 configured to identify one or more oral health issues in the dental images 104, and an application 114. The machine-learning model 108 can be integrated into the application 114 or can be a separate entity on the end user device 102. In an alternative embodiment, the machine-learning model 108 can execute on a remote computing device and communicates with the end user device 102 over suitable communication networks. FIG. 1 also shows a mouth map 106, which will be illustrated in more detail further below.

The dental images 104 used in embodiments can comprise various imaging modalities, formats, perspectives, and/or technical variants used in clinical settings, including without limitation:
- Radiographic (X-ray based) images, such as intraoral X-ray images (e.g., bitewing X-ray, periapical X-ray, occlusal X-ray, or the like), extraoral X-ray images (e.g., panoramic radiograph, cephalometric radiograph, posteroanterior skull view, submentovertex view, Waters view, or reverse Towne radiograph), digital radiographs (e.g., digital intraoral X-ray, digital panoramic image, or digital cephalometric image), and contrast-enhanced X-ray images (e.g., sialography or contrast-based TMJ imaging), orthopantomography (OPG) X-ray images, or the like.
- Tomographic images, such as computed tomography (CT) images (e.g., cone beam CT, dental CT scan, multi-slice spiral CT, or the like), tomographic slice views (e.g., axial, sagittal, or coronal CT slices), and 3D renderings based on CT data, or the like.
- Magnetic resonance images (MRI), such as dental MRI images, TMJ MRI images, soft-tissue MRI scans, or functional MRI (fMRI) scans, or the like.
- Optical and photographic images, such as intraoral photographs (e.g., buccal, lingual, occlusal, or frontal views), extraoral photographs (e.g., profile, frontal face at rest or smiling, chin-up, or chin-down views), intraoral camera images, microscope images (e.g., from a dental operating microscope), fluorescence-based images (e.g., using QLF or DIAGNOdent systems), and transillumination images (e.g., FOTI or DIFOTI), or the like.
- Motion-based and 3D images, such as videos of the oral cavity (e.g., intraoral video, endoscopic video, or time-lapse video), intraoral scanner video captures, 3D surface scans (e.g., intraoral digital impressions, 3D facial scans, or scanned dental models), and optical coherence tomography (OCT) images, or the like.
- Histological and microscopic images, such as histological section images of dental tissue, scanning electron microscopy (SEM) images, confocal microscopy images, and polarized light microscopy images, or the like.
- Specialty and diagnostic images, such as thermal images (e.g., oral thermography), laser Doppler images for pulp vitality, spectral or hyperspectral images of dental tissue, autofluorescence images, cariogenic biofilm images, plaque-disclosing solution images, orthodontic treatment progress images, or surgical planning images (e.g., for dental implant positioning), or the like.
- Synthetic and derived images, such as AI-synthesized dental images, segmentation masks, annotated image overlays (e.g., with tooth labels or issue markers), 3D model renders from scan data, or attention heatmaps for explainable AI analysis, or the like.

The oral health issues identified in the dental images 104 in embodiments can comprise, without limitation:
- Dental health issues, such as cavities, dental caries (e.g., incipient, enamel, dentinal, or root caries), periodontal disease (e.g., gingivitis, periodontitis, bone loss, or periodontal pockets), endodontic conditions (e.g., periapical abscess, pulpitis, pulp necrosis, or root canal infections), and tooth fractures (e.g., crown fracture, root fracture, or cracked tooth syndrome), or the like.
- Tooth anomalies, such as impactions (e.g., impacted third molars or canines), missing teeth (e.g., congenitally missing or extracted teeth), supernumerary teeth (e.g., mesiodens), ectopic eruptions, or retained deciduous teeth, or the like.
- Malocclusions and orthodontic findings, such as crowding, spacing, overjet, overbite, open bite, crossbite, midline shift, and class I, II, or III skeletal or dental relationships, or the like.
- Temporomandibular joint (TMJ) disorders, such as condylar degeneration, joint effusion, articular disc displacement, condylar asymmetry, ankylosis, or arthritic changes, or the like.
- Periapical and alveolar findings, such as periapical radiolucencies (e.g., granuloma or cyst), widened periodontal ligament space, lamina dura discontinuity, and alveolar bone density anomalies, or the like.
- Dental restorations and materials, such as defective restorations (e.g., overhangs or recurrent decay), crowns, bridges, inlays, onlays, dental fillings, root canal fillings, posts, and implants (e.g., peri-implantitis or implant failure), or the like.
- Developmental anomalies and syndromic findings, such as taurodontism, dens invaginatus, enamel hypoplasia, dentinogenesis imperfecta, amelogenesis imperfecta, cleft palate involvement, or other craniofacial malformations, or the like.
- Oral pathology findings, such as benign lesions (e.g., fibroma, papilloma), cysts (e.g., radicular, dentigerous, or keratocystic odontogenic tumors), malignant tumors (e.g., squamous cell carcinoma, osteosarcoma), mucosal abnormalities (e.g., leukoplakia, erythroplakia), and bone pathologies (e.g., osteomyelitis, osteonecrosis, or fibro-osseous lesions), or the like.
- Sinus and adjacent structure conditions, such as maxillary sinusitis, oroantral communication, sinus floor thickening, or sinus perforation from dental pathology or procedures, or the like.
- Orthodontic appliance-related findings, such as presence or displacement of brackets, archwires, mini-implants (TADs), expanders, retainers, or signs of appliance-related root resorption, or the like.
- Trauma-related findings, such as avulsed teeth, luxation injuries (e.g., intrusion, extrusion, lateral displacement), alveolar fracture, or foreign bodies in the oral cavity, or the like.
- Implant-related findings, such as implant placement accuracy, osseointegration status, peri-implant radiolucency, or surrounding bone loss indicative of implant failure, or the like.
- Surgical planning or post-op findings, such as bone graft volume, sinus lift evaluation, nerve canal proximity, healing progression after extraction or surgery, or complications such as dry socket or infection, or the like.

FIG. 2 illustrates a flowchart of a method 200 of providing personalized oral care guidance in accordance with one embodiment. The method 200, or individual steps thereof, may be performed by the end user device 102 shown in FIG. 1. In image reception step 202, method 200 receives a dental image 104 of the subject 110 at the end user device 102 of the subject 110, the dental image 104 being captured by an oral scanning device. In issue identification step 204, method 200 identifies one or more oral health issues in the dental image 104 using image-processing machine-learning model 108. In mouth map generation step 206, method 200 generates a personalized mouth map 106 of the subject. The mouth map 106 indicates the one or more oral health issues identified in the previous step. In displaying step 208, method 200 displays the mouth map 106 with the indicated one or more oral health issues on a display of the end user device 102.

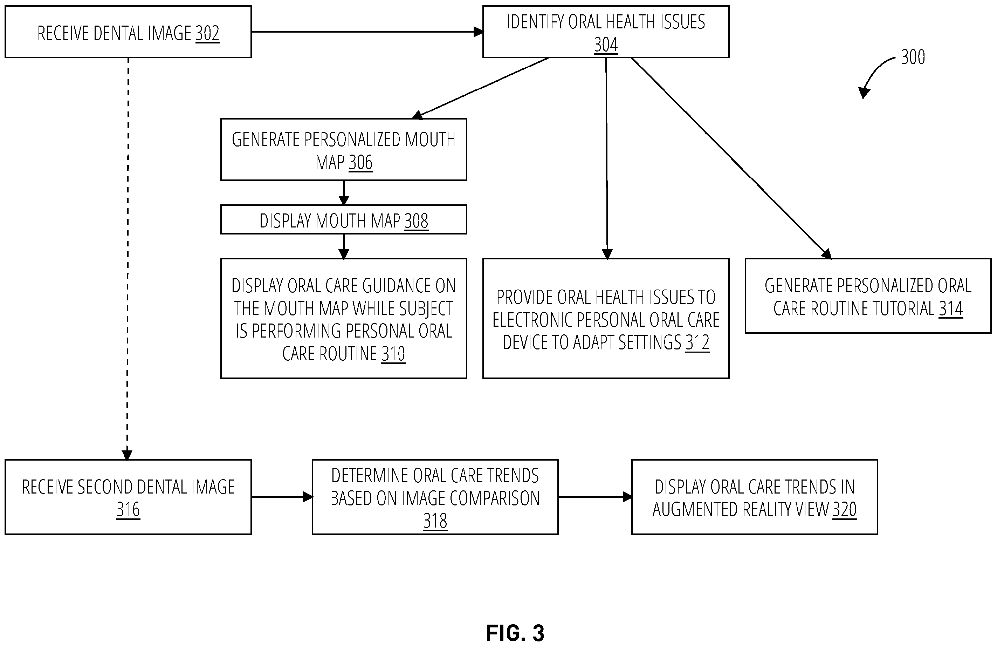

FIG. 3 illustrates a more detail flowchart of a method 300 of providing personalized oral care guidance in accordance with one embodiment. The method 300, or individual steps thereof, may be performed by the end user device 102 shown in FIG. 1. Method 300 represents a complete embodiment that combines several unique functionalities disclosed herein. Alternative embodiments can include only suitable sub-combinations of the discussed functionalities.

In a first image reception step 302, which is similar to image reception step 202 in FIG. 2, method 300 receives a first dental image 104 of the subject 110 at the end user device 102 of the subject 110, the first dental image 104 being captured by an oral scanning device. For example, the subject 110 can upload an image, video, x-ray report, or the like, into the application 114 on the end user device 102. The first dental image 104 provides a detailed view of the subject 110's teeth, gums, jaw and tongue, and can reveal any dental issues such as cavities, gum disease or misalignment.

In issue identification step 304, which is similar to issue identification step 204 in FIG. 2, method 300 identifies one or more oral health issues in the first dental image 104 using image-processing machine-learning model 108. The machine-learning model 108 may analyze the first dental image 104 to generate a detailed report of the subject 110's tooth and/or dental conditions.

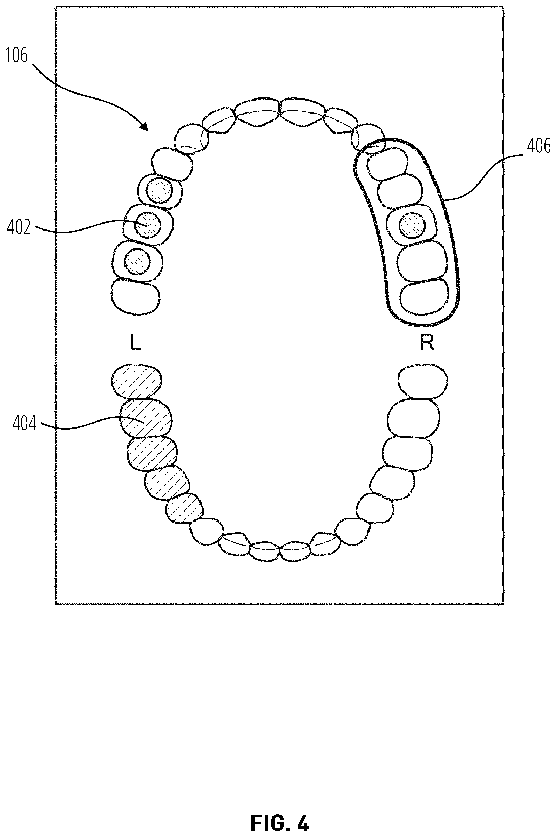

In mouth map generation step 306, which is similar to mouth map generation step 206 in FIG. 2, method 300 generates a personalized mouth map 106 of the subject 110. The report may be used to create a personalized mouth map 106, ideally tooth by tooth, in the application 114. An exemplary mouth map 106 will be discussed in connection with FIG. 4 below.

In displaying step 308, which is similar to displaying step 208 in FIG. 2, method 300 displays the mouth map 106 with the indicated one or more oral health issues on a display of the end user device 102.

In live guidance displaying step 310, method 300 displays oral care guidance on the mouth map 106 while the subject 110 is performing a personal oral care routine. Accordingly, the application 114 can use the personalized mouth map 106 to guide the subject 110 in real time as they brush their teeth. The application 114 can highlight the areas of focus on the mouth map 106 and provide live feedback to ensure that the subject 110 is brushing these areas effectively. The focus areas need not be manually entered by the subject 110, but are auto-derived from the dental image(s) using the techniques described in this disclosure. This integration allows for a highly personalized and effective brushing experience, tailored to the subject 110's specific dental health needs. This live guidance functionality can be implemented separately from the other unique features disclosed herein.

In device control step 312, method 300 provides the one or more oral health issues to the personal oral care device of the subject 110 to adapt one or more settings thereof. For example, the report generated by the machine-learning model 108, containing detailed insights at the subsegment level, can be provided to the subject's 110 toothbrush. Using this data, the toothbrush can automatically adjust its brushing techniques for each subsegment to ensure effective cleaning. In one implementation example, the settings per segment and/or tooth location can be sent to the personal oral care device so that the device can just apply them. In another implementation example, a report can be sent, based on which the personal oral care device derives the settings. This device control functionality can be implemented separately from the other unique features disclosed herein.

In tutorial generating step 314, method 300 generates a personalized oral care routine tutorial for the subject 110. This way, the subject 110 is effectively taught the right routine personalized to their situation. This tutorial generation functionality can be implemented separately from the other unique features disclosed herein.

In a second image reception step 316, method 300 receives a second dental image 104. The expressions "first" and "second" merely serve to indicate that an initial dental image 104, namely the "first" one, has already been processed as described above before a subsequent dental image 104, namely the "second" one, is received. The following functionalities may be repeated every time a subsequent dental image 104 is received, i.e., a "third" dental image 104, a "fourth" dental image 104, and so on. The second, subsequent dental image(s) 104 may originate from one or more subsequent dentist visits by the subject 110. As already mentioned elsewhere, instead of dental images 104 captured by a dentist, also the subject 110 themselves may capture such dental images 104, e.g., by using a home oral scanning device.

In trend determination step 318, method 300 determines at least one oral care trend based on a comparison of the first dental image 104 and the second dental image 104. In trend display step 320, method 300 displays the at least one oral care trend. Accordingly, upon uploading a new dental image 104 to the application 114, the machine-learning model 108 analyzes the data to identify trends, highlight areas of improvement and pinpointing regions that require attention. In a preferred implementation, the application 114 leverages Augmented Reality (AR) to make the comparison of the report visually engaging and easily understandable for the subject 110. In addition or alternatively to specific oral care trends, the displaying may also comprise a side-by-side comparison of the first and second dental image 104. Optionally, based on the identified trend(s), feedback can be given to dental insurance providers who can use it for insurance plan adjustments and/or provide incentives based on the progress and/or improvements. This eventually gives the subject 110 another inventive to improve their oral hygiene.

FIG. 4 illustrates an example of a mouth map on a graphical user interface in accordance with one embodiment. As can be seen, the mouth map 106 is a visual representation of the subject 110's mouth. In a preferred implementation, the mouth map 106 is customized to the subject 110's teeth, e.g., indicating any gaps, missing teeth, or other individual oral characteristics derivable from the dental image 104. A focus area 406 that needs special attention based on the findings of the machine-learning model 108 is shown in the mouth map 106. The mouth map 106 also indicates several cavities 402 and an inflamed gum 404 as examples of oral health issues.

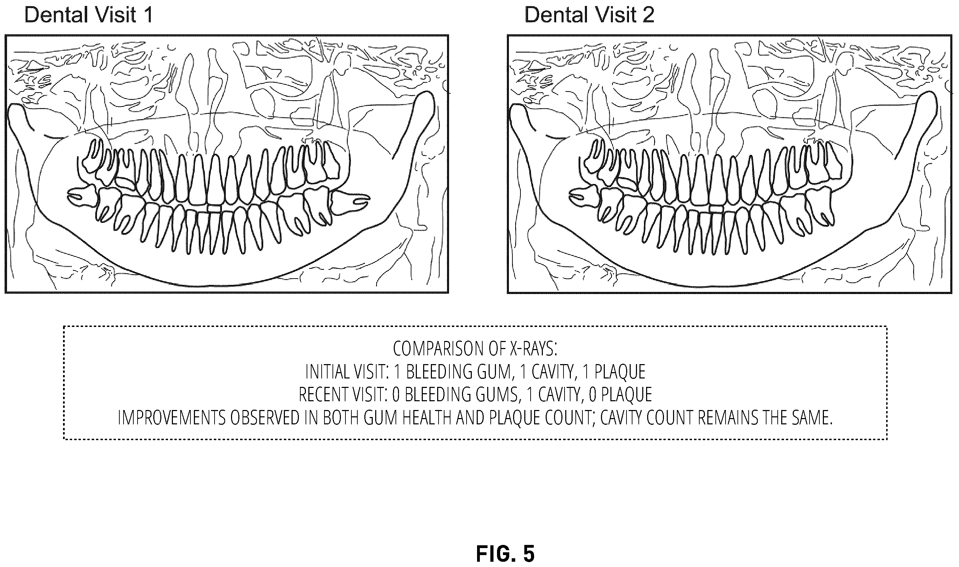

FIG. 5 illustrates an example of an augmented reality view of a comparison of dental images in accordance with one embodiment. As can be seen, the augmented reality view shows a first dental image 104, in this case an orthopantomography (OPG) X-ray image, captured during a first dental visit on the left, and a second dental image 104, also an OPG X-ray image, captured during a second, later dental visit on the right. The interactive augmented reality makes the comparison visually engaging and easily understandable for the subject. In addition, the augmented reality view shows oral care trends based on the comparison, in the illustrated example "Initial visit: 1 bleeding gums, 1 cavities, 1 Plaque", "Recent visit: 0 bleeding gums, 1 cavities, 0 Plaque", and "Improvements observed in both gum health and plaque count; cavity count remains the same". Based on the progress and/or improvements determined from the comparison of the dental images, feedback can be given to dental insurance providers who can use it for insurance plan adjustments and/or provide incentives based on the progress and/or improvements. This eventually gives the subject another inventive to improve their oral hygiene.

In various embodiments, the machine-learning model 108 may be an image-processing machine-learning model that is configured to analyze dental images 104 of a subject's teeth and to automatically identify one or more oral health issues. In the following, several concrete examples for implementing such a machine-learning model 108 will be described:
In a first example implementation, the machine-learning model 108 comprises a convolutional neural network (CNN), optionally implemented as a deep residual network (ResNet) or a U-Net architecture, tailored for high-precision image recognition tasks in the dental domain. The CNN may include an input layer configured to accept grayscale images of a fixed resolution (e.g., 512×512 pixels), multiple convolutional layers with kernel sizes ranging from 3×3 to 7×7, and between 16 and 128 filters per layer, followed by ReLU activation functions and max-pooling layers for spatial downsampling. Batch normalization and dropout layers may be included to improve generalization and prevent overfitting. The output layer may include a dense layer with sigmoid or softmax activation to classify the presence and location of one or more dental conditions, such as the probability of caries in each tooth region.

In a second example implementation, the machine-learning model 108 comprises a transformer-based vision model, such as a Vision Transformer (ViT) or a Swin Transformer. In this configuration, the machine-learning model 108 uses self-attention mechanisms rather than convolutional layers. In one exemplary implementation, the input image is first divided into fixed-size non-overlapping patches (e.g., 16×16 pixels), which are then flattened and linearly embedded into patch tokens. Positional encodings may be added to preserve spatial information. The embedded tokens are then passed through a stack of transformer encoder blocks, each comprising multi-head self-attention layers and feedforward neural networks with GELU activation functions. The model may include between 8 and 24 attention heads and 12 to 32 encoder layers, depending on the desired accuracy and available computational resources. The final output of the transformer model is processed via a classification head, such as a multilayer perceptron (MLP), to predict the presence and location of specific oral health conditions. This architecture is particularly advantageous for capturing long-range dependencies and complex anatomical structures in panoramic or 3D dental images.

In a third example implementation, the machine-learning model 108 is specifically configured to process temporal sequences of dental images, such as time-series radiographs captured across multiple visits. The model may comprise a hybrid architecture that combines convolutional neural networks (CNNs) with recurrent neural networks (RNNs), such as long short-term memory (LSTM) networks. In this implementation, each individual dental image in the sequence is first processed by a CNN backbone-e.g., a MobileNet or EfficientNet model-that extracts spatial features. The resulting feature vectors for each time step are then sequentially passed into one or more stacked LSTM layers to capture temporal patterns and disease progression. The LSTM layers may include between 64 and 256 hidden units and may be followed by fully connected layers with dropout regularization. This hybrid model architecture allows for the identification of evolving oral health conditions, such as the progression of periodontal disease or the development of caries over time, thereby enabling predictive diagnostics based on historical imaging data.

In a fourth example implementation, a training/validation dataset was created. To this end, images having cavities, gum diseases, missing teeth and misalignment were collected. The images were annotated with oral health issues using a computer vision tool called Roboflow, labeling objects as "Cavity_1", "Cavity_2", "Gum_1", etc. After annotation, Roboflow provided a ZIP file containing all images and a JSON file with annotations. For the model training, a tool for training machine-learning models called Create ML was used to fine-tune a pre-defined object detection model. The dataset was split into 80% training and 20% validation to ensure proper learning and evaluation. The trained machine-learning model was exported as a .mlmodel file. The .mlmodel file was then integrated into an iOS mobile application. The iOS application used the machine-learning model to detect dental issues and to highlight focus areas for further analysis.

While each of the exemplary implementations of the machine-learning model 108 may put an emphasize on certain characteristics of the disclosed methods, it is apparent that these implementations are only provided as examples for carrying out the concepts disclosed herein, but none of these implementation details is essential for the disclosed concepts.

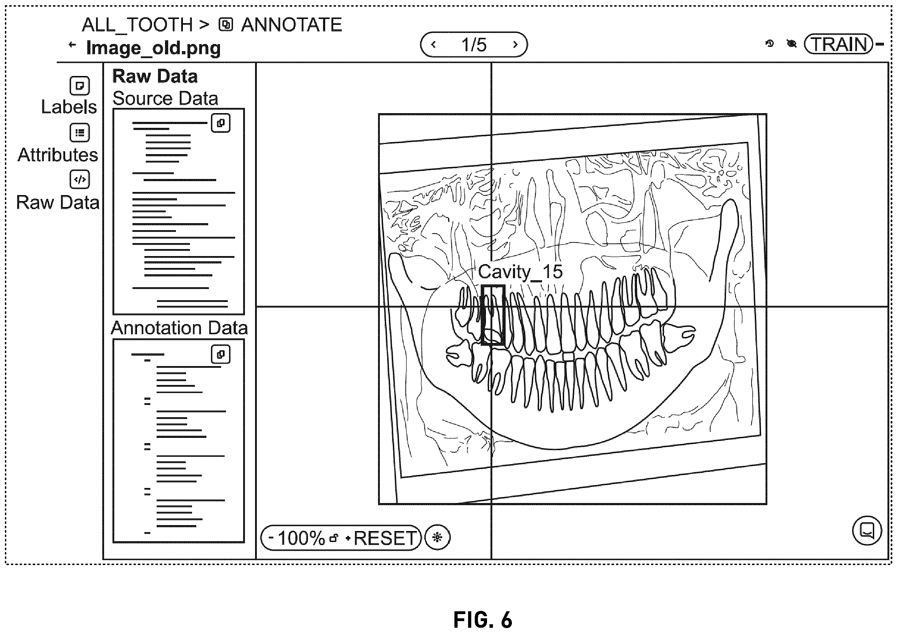

Regardless of its exact implementation, the machine-learning model 108 can be trained in a supervised manner using a curated dataset comprising annotated dental images, in particular with a plurality of dental images labelled with oral health issues. An example of such a labeled training image in accordance with one embodiment is shown in FIG. 6, in which a specific tooth in an X-ray image has been labelled "Cavity_15", wherein "15" uniquely identifies the tooth according to the Universal Numbering System adopted by the American Dental Association, and "Cavity" indicates that this tooth contains a cavity. Other types of tooth identification notations could be used as well, such as the Palmer Notation Method, the FDI World Dental Federation notation, or the like. Each training image can be labeled with bounding boxes, segmentation masks, or keypoints indicating the location and type of diagnosed oral health issues. These annotations may be provided by licensed dental professionals. During training, the machine-learning model 108 can minimize a loss function such as binary cross-entropy or focal loss for classification tasks and a combination of Dice coefficient loss and cross-entropy for segmentation tasks. The training process may employ backpropagation and stochastic gradient descent or adaptive optimizers (e.g., Adam) with suitable hyperparameters (e.g., learning rate of 0.001, batch size of 32, training over 50-100 epochs). The trained machine-learning model 108 can be deployed on the end user device 102.

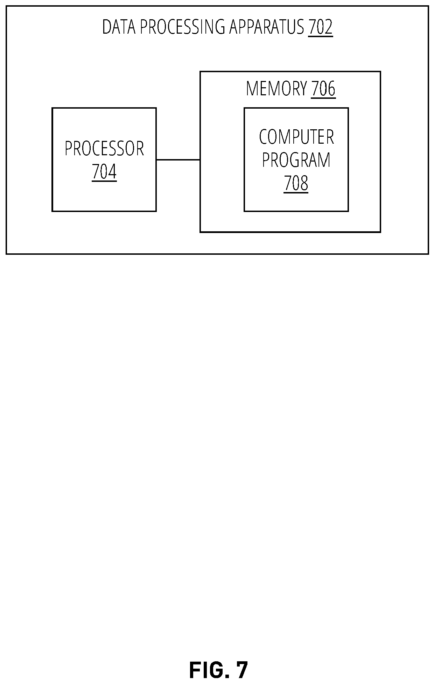

FIG. 7 illustrates a schematic block diagram of computer hardware on which embodiments of the present disclosure can be implemented. As can be seen, a data processing apparatus 702 comprises one or more processors 704 and a memory 706. The one or more processors 704 are communicatively coupled to the memory 706. The memory 706 stores a computer program 708. The computer program 708 may implement some or all aspects of the disclosed methods and functionalities.

With certain embodiments presented herein, a novel application is provided that leverages the power of X-ray imaging, Augmented Reality (AR), and Artificial Intelligence (AI) to provide personalized dental care guidance to users. In one exemplary specific implementation, the application begins by importing a user's dental X-ray, which is used to create a customized mouth map. This map identifies areas of focus for dental care based on the user's unique dental health profile. As the user brushes their teeth, the application provides live guidance to ensure effective cleaning in the focus areas. This real-time feedback is made possible through the use of AR, creating an engaging and interactive user experience. After a subsequent dental visit, a new X-ray is taken and uploaded to the application. The application then uses AI to compare the two X-rays, identifying areas of improvement and areas that still require attention. This comparison is presented to the user in an interactive AR format, allowing them to visually understand their progress and areas of focus. By providing personalized, real-time feedback and tracking progress over time, this application empowers users to take control of their dental health in a way that is engaging and easy to understand.

It is apparent to those skilled in the art that embodiments of the present disclosure as presented above can produce one or more of the following advantages:
- Personalized Dental Care: The application provides personalized dental care advice based on the subject's unique dental health profile, as identified through image analysis.
- Improved Brushing Techniques: With the personal oral care device's auto-adaptation feature and live, interactive guidance during brushing, the application empowers subjects to refine their brushing habits, ensuring thorough cleaning of all areas in the mouth.
- Dental Health Tracking: The application allows subjects to track their dental health progress over time. By comparing different dental images taken at different times, subjects can see how their dental health has improved or identify areas that need more attention.
- Education and Understanding: The application can use augmented reality to help subjects better understand their dental health and the importance of good dental hygiene. This can lead to improved dental care habits and overall better dental health.
- Increased Engagement: The interactive nature of the application can make dental care more engaging, encouraging subjects to take a more active role in their dental health.

While various aspects and embodiments have been illustrated and described in detail in the foregoing description and the drawings, such illustration and description is illustrative or exemplary and not restrictive. Variations to the disclosed embodiments can be understood and effected by those skilled in the art in practicing the claimed subject-matter, from a study of the drawings, the disclosure, and the appended claims.

Although some aspects have been described in the context of a product, apparatus, device or system, these aspects also represent a description of the corresponding process, method or use, where a block or component corresponds to a method step or a feature of a method step. Analogously, aspects described in the context of a method step also represent a description of a corresponding block or component or feature of a corresponding product, apparatus, device or system.

The order of execution of the operations in the described embodiments is not essential, unless otherwise specified. That is, the operations may be performed in any order, unless otherwise specified, and embodiments may include additional or fewer operations than those mentioned.

In the claims, the word "comprising" does not exclude other elements or steps, and the indefinite article "a" or "an" does not exclude a plurality. A single entity may fulfill the functions of several entities recited in the claims. The mere fact that certain measures are recited in mutually different dependent claims does not indicate that a combination of these measures cannot be used to advantage. Any reference signs in the claims should not be construed as limiting the scope.

Embodiments of the present disclosure can be implemented in hardware, software, or both. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer-readable.

Embodiments of the present disclosure can be implemented on a computer system. The computer system may be a local computer device (e.g., personal computer, laptop, tablet computer or mobile phone) with one or more processors and one or more storage devices or may be a distributed computer system (e.g., a cloud computing system with one or more processors and one or more storage devices distributed at various locations, for example, at a local client and/or one or more remote server farms and/or data centers). The computer system may comprise any circuit or combination of circuits. In one embodiment, the computer system may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), or any other type of processor or processing circuit. Other types of circuits that may be included in the computer system may be a custom circuit, an application-specific integrated circuit (ASlC), or the like, such as, for example, one or more circuits (such as a communication circuit) for use in wireless devices like mobile telephones, tablet computers, laptop computers, two-way radios, and similar electronic systems. The computer system may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system may also include a display device, one or more speakers, and a keyboard and/or controller, which can include a mouse, trackball, touch screen, voice-recognition device, or any other device that permits a system user to input information into and receive information from the computer system. Some or all of the method steps may be executed by (or using) a hardware apparatus, like for example, a processor, a microprocessor, a programmable computer or an electronic circuit. In some embodiments, some one or more of the described method steps may be executed by such an apparatus. A further embodiment is an apparatus as described herein comprising a processor and a storage medium.

Embodiments of the present disclosure can be implemented as a computer program (product) with a program code, the program code being operative for performing one of the methods when the computer program product runs on a computer. The program code may, for example, be stored on a machine-readable carrier. Other embodiments comprise a computer program for performing one of the methods described herein, stored on a machine-readable carrier. A further embodiment is a computer program having a program code for performing one of the methods described herein, when the computer program runs on a computer. A further embodiment is a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitory. A further embodiment is a computer having installed thereon the computer program for performing one of the methods described herein, or individual steps thereof.

A further embodiment is a data stream or a sequence of signals representing the computer program for performing one of the methods described herein. The data stream or the sequence of signals may, for example, be configured to be transferred via a data communication connection, for example, via the internet.

A further embodiment is an apparatus or a system configured to transfer (e.g., electronically or optically) a computer program for performing one of the methods described herein to a receiver. The receiver may, for example, comprise a computer, a mobile device, a memory device or the like. The apparatus or system may, for example, comprise a file server for transferring the computer program to the receiver.

### REFERENCE SIGNS

102 end user device
104 dental image
106 mouth map
108 machine-learning model
110 subject
112 personal oral care device
114 application
116 dentist
200 method
202 image reception step
204 issue identification step
206 mouth map generation step
208 displaying step
300 method
302 first image reception step
304 issue identification step
306 mouth map generation step
308 displaying step
310 live guidance displaying step
312 device control step
314 tutorial generating step
316 second image reception step
318 trend determination step
320 trend display step
402 cavity
404 gum disease
406 focus area
702 data processing apparatus
704 processor
706 memory
708 computer program

## Claims

1. A method of providing personalized oral care guidance, the method comprising:
receiving a dental image (104) of a subject (110) at an end user device (102) of the subject (110), the dental image (104) being captured by an oral scanning device;
identifying one or more oral health issues in the dental image (104) using an image-processing machine-learning model (108);
generating a mouth map (106) of the subject (110) which indicates the one or more oral health issues; and
displaying the mouth map (106) with the indicated one or more oral health issues on a display of the end user device (102).

2. The method of claim 1, wherein the one or more oral health issues comprise one or more of:
an indication of a cavity (402);
an indication of plaque build-up;
an indication of a gum disease (404);
an indication of a misalignment;
an indication of tongue hygiene.

3. The method of claim 1 or 2, further comprising:
displaying oral care guidance on the mouth map while the subject is performing a personal oral care routine;
wherein the oral care guidance comprises one or more focus areas (406).

4. The method of any one of claims 1 to 3, further comprising:
providing the one or more oral health issues to an electronic personal oral care device to cause the personal oral care device to adapt at least one oral care setting.

5. The method of any one of claims 1 to 4, further comprising:
generating a personalized oral care routine tutorial based on the one or more oral health issues.

6. The method of any one of claims 1 to 5, wherein the dental image is a first dental image, and wherein the method further comprises:
receiving a second dental image of the subject which was taken later than the first dental image;
determining at least one oral care trend based on a comparison of the first dental image and second dental image; and
displaying the at least one oral care trend in an augmented reality view.

7. The method of any one of claims 1 to 6, wherein the first dental image and/or the second dental image comprises one or more of:
an X-ray image;
a CT image;
an MRI image;
a photo;
a video.

8. The method of any one of claims 1 to 7, wherein the first dental image and/or the second dental image show the subject's teeth, gums, jaw and tongue.

9. The method of any one of claims 1 to 8, wherein the machine-learning model has been trained on a training dataset comprising a plurality of dental training images labelled with oral health issues.

10. The method of any one of claims 1 to 9, wherein the machine-learning model executes on the end user device; or
wherein the machine-learning model executes on a computing device remote from the end user device.

11. The method of any one of claims 1 to 10, wherein the mouth map comprises a visual representation of the subject's mouth.

12. The method of any one of claims 1 to 11, wherein the mouth map is customized to the subject's mouth.

13. The method of any one of claims 1 to 12, wherein the mouth map is a tooth-by-tooth mouth map.

14. A data processing apparatus, in particular an end user device, comprising means for carrying out the method of any one of claims 1 to 13.

15. A computer program or a computer-readable medium storing a computer program, the computer program comprising instructions which, when the program is executed on a computer, cause the computer to carry out the method of any one of claims 1 to 13.
